# EUROPEAN PATENT APPLICATION

(11) **EP 4 491 744 A1**
(43) Date of publication of application: **15.01.2025**
(21) Application number: 23879722.9
(22) Date of filing: 13.10.2023
(51) Int. Cl.: C12Q 1/6883, C12N 15/12, C12Q 1/6827, C12Q 1/686

(54) **PRIMER SET, KIT FOR DETERMINING ONSET AND/OR ONSET RISK OF SPINAL MUSCULAR ATROPHY, AND METHOD FOR DETERMINING ONSET AND/OR ONSET RISK OF SPINAL MUSCULAR ATROPHY**

(30) Priority: 17.10.2022 JP 2022166030
(71) Applicant: Craif Inc., Tokyo 113-0034 (JP)
(72) Inventor: HIRANO, Masaki, Nagoya-shi, Aichi 466-0807 (JP)
(74) Representative: Michalski Hüttermann & Partner Patentanwälte mbB
(86) International application number: PCT/JP2023/037160
(87) International publication number: WO 2024/085079

(57) **Abstract**

[Object] Since the SMN2 gene close to the SMN1 gene is different from the SMN1 gene only by five bases, an inspection system that can specifically detect only the SMN1 gene with high sensitivity needs to be constructed for an SMA screening test.

[Solution] Provided are a primer set that amplifies a nucleic acid encoding survival motor neuron (SMN) 1 and does not amplify a nucleic acid encoding survival motor neuron (SMN) 2, a kit including the primer set for determining whether or not a subject has developed spinal muscular atrophy and/or a risk of developing spinal muscular atrophy, and a method for determining whether or not a subject has developed spinal muscular atrophy and/or a risk of developing spinal muscular atrophy by using the primer set.

## Description

### TECHNICAL FIELD

The present invention relates to a primer set that amplifies a nucleic acid encoding survival motor neuron (SMN) 1 and does not amplify a nucleic acid encoding survival motor neuron (SMN) 2, a kit including the primer set for determining whether or not a subject has developed spinal muscular atrophy and/or a risk of developing spinal muscular atrophy, and a method for determining whether or not a subject has developed spinal muscular atrophy and/or a risk of developing spinal muscular atrophy by using the primer set.

### BACKGROUND ART

The spinal muscular atrophy (SMA) is neurogenic muscular atrophy caused by degeneration of motor nerve cells of a spinal cord (spinal anterior horn cells) and is classified into a motor neuron disease. The SMA is classified into plural types depending on an age of onset and a symptom, and type I whose symptoms appear within about six months from birth is likely to remain undiagnosed for a long time and result in severe symptoms.

Since it is known that almost all SMA patients have homozygous deletion of the Survival of Motor Neuron 1 (SMN1) gene, a screening test aimed at the SMN1 gene is effective for early discovery of an SMA risk. Those who have heterozygous deletion of the SMN1 gene are called carriers since their descendants have a risk of homozygous deletion. The screening test is also required to be able to determine whether or not a subject is an SMA carrier.

As techniques related to an SMA screening test using a molecular biological technique such as PCR, techniques such as a method for specifically detecting and quantifying the SMN1 gene included in a punch piece of a certain size obtained from a dried blood spot by direct real-time PCR and primers used for the method (Patent Literature 1), a primer set suitable for an SMA screening test and a probe for real-time PCR (Non Patent Literatures 1 to 3) are known.

### CITATION LIST

### PATENT LITERATURE

PTL 1: Japanese Patent No. 6850402

### NON PATENT LITERATURE

NPL 1: Vill, Katharina et al., Journal of Neuromuscular Diseases, 2019
NPL 2: Pan, Jianyan et al., Annals of Laboratory Medicine, 2022
NPL 3: Feldkotter, Markus et al., American Journal of Human Genetics, 2002

### SUMMARY OF INVENTION

### TECHNICAL PROBLEM

Since the SMN2 gene close to the SMN1 gene is different from the SMN1 gene only by five bases, a method that can specifically detect only the SMN1 gene with high sensitivity needs to be constructed for an SMA screening test.

According to inventors' studies, amplification efficiency which the primer sets described in the conventional art literatures exhibit in a case where a DNA sample without an SMN1 gene deletion is used as a template is significantly low as compared with the RPPH1 gene and the RPP20 gene used as internal standards and is almost identical to or slightly higher than that in a case where a DNA sample with homozygous deletion and/or heterozygous deletion is used as a template. Therefore, according to the techniques described in the conventional art literatures, it is not easy to distinguish DNA without deletion and DNA with homozygous deletion and/or heterozygous deletion by using a Ct value obtained by real-time PCR as an index. Furthermore, according to the techniques described in the conventional art literatures, it is extremely difficult to determine whether or not a subject is an SMA patient by electrophoretic analysis or lateral flow assay.

### SOLUTION TO PROBLEM

Specifically, the present invention is a primer set for determining whether or not a subject has developed spinal muscular atrophy and/or a risk of developing spinal muscular atrophy, in which the primer set amplifies a Survival motor neuron (SMN) 1 gene, and a forward primer and a reverse primer included in the primer set each have a base that hybridizes to a base on the SMN1 gene that differs in a case where the SMN1 gene is aligned with a SMN2 gene.

In one aspect of the present invention, the primer set is arranged such that the bases of the forward primer and the reverse primer are located on 3' end sides of the forward primer and the reverse primer. In another aspect of the present invention, the primer set can be arranged such that the bases of the forward primer and the reverse primer are located at any of first to fifth positions from 3' ends of the forward primer and the reverse primer.

In one aspect of the present invention, the primer set can be arranged such that the base of the forward primer hybridizes to a base complementary to any of 152nd, 201st, 349th, and 464th bases of a base sequence indicated by SEQ ID No. 1, and the base of the reverse primer hybridizes to a base homologous to any of the 201st, 349th, 464th, and 929th bases of the base sequence indicated by SEQ ID No. 1.

In another aspect of the present invention, the primer set can be arranged such that the forward primer is any of (F1) to (F4), and the reverse primer is any of (R1) to (R4).
(F1) a primer that has, at any of first to fifth positions from a 3' end, a base homologous to a 152nd base of a base sequence indicated by SEQ ID No. 1 and has, in a 5' direction from the base, a sequence consecutively homologous to a base sequence in a 5' direction from the 152nd base of the base sequence indicated by SEQ ID No. 1
(F2) a primer that has, at any of first to fifth positions from a 3' end, a base homologous to a 201st base of the base sequence indicated by SEQ ID No. 1 and has, in a 5' direction from the base, a sequence consecutively homologous to a base sequence in the 5' direction from the 201st base of the base sequence indicated by SEQ ID No. 1
(F3) a primer that has, at any of first to fifth positions from a 3' end, a base homologous to a 349th base of the base sequence indicated by SEQ ID No. 1 and has, in a 5' direction from the base, a sequence consecutively homologous to a base sequence in the 5' direction from the 349th base of the base sequence indicated by SEQ ID No. 1
(F4) a primer that has, at any of first to fifth positions from a 3' end, a base homologous to a 464th base of the base sequence indicated by SEQ ID No. 1 and has, in a 5' direction from the base, a sequence consecutively homologous to a base sequence in the 5' direction from the 464th base of the base sequence indicated by SEQ ID No. 1
(R1) a primer that has, at any of first to fifth positions from a 3' end, a base complementary to the 201st base of the base sequence indicated by SEQ ID No. 1 and has, in a 5' direction from the base, a sequence consecutively complementary to a base sequence in a 3' direction from the 201st base of the base sequence indicated by SEQ ID No. 1
(R2) a primer that has, at any of first to fifth positions from a 3' end, a base complementary to the 349th base of the base sequence indicated by SEQ ID No. 1 and has, in a 5' direction from the base, a sequence consecutively complementary to a base sequence in the 3' direction from the 349th base of the base sequence indicated by SEQ ID No. 1
(R3) a primer that has, at any of first to fifth positions from a 3' end, a base complementary to the 464th base of the base sequence indicated by SEQ ID No. 1 and has, in a 5' direction from the base, a sequence consecutively complementary to a base sequence in the 3' direction from the 464th base of the base sequence indicated by SEQ ID No. 1
(R4) a primer that has, at any of first to fifth positions from a 3' end, a base complementary to a 929th base of the base sequence indicated by SEQ ID No. 1 and has, in a 5' direction from the base, a sequence consecutively complementary to a base sequence in the 3' direction from the 929th base of the base sequence indicated by SEQ ID No. 1

In another aspect of the present invention, the primer set can be arranged such that the forward primer and the reverse primer are one or more selected from the group consisting of the following (a) to (d).
(a) the forward primer is the (F1), and the reverse primer is any of the (R1) to (R4)
(b) the forward primer is the (F2), and the reverse primer is any of the (R2) to (R4)
(c) the forward primer is the (F3), and the reverse primer is the (R3) or the (R4)
(d) the forward primer is the (F4), and the reverse primer is the (R4)

In another aspect of the present invention, the primer set can be arranged such that the forward primer and/or the reverse primer have a mismatch base that forms a mismatched base pair with the SMN1 gene. The mismatch base may be adjacent to the base that hybridizes to the base on the SMN1 gene that differs in a case where the SMN1 gene is aligned with the SMN2 gene.

In another aspect of the present invention, the primer set can be a primer set that specifically amplifies the SMN1 gene, and does not amplify the SMN2 gene or hardly amplifies the SMN2 gene.

In another aspect of the present invention, the primer set can be arranged such that the forward primer and the reverse primer have a length of 8 to 40 bases.

In another aspect of the present invention, the primer set can be arranged such that the forward primer is any of (F5) to (F7), and the reverse primer is any of (R5) to (R7).
(R5) a nucleic acid having a base sequence indicated by any of SEQ ID Nos. 3 to 18, SEQ ID Nos. 35 to 41, SEQ ID Nos. 49 to 56, and SEQ ID No. 75
(R6) a nucleic acid having a base sequence obtained by deletion, substitution, addition, and/or insertion of one or more bases in the base sequence described in (F5)
(F7) a nucleic acid that hybridizes to a nucleic acid having a base sequence complementary to the base sequences described in (F5) and (F6) under a stringent condition
(R5) a nucleic acid having a base sequence indicated by any of SEQ ID Nos. 19 to 34, SEQ ID Nos. 42 to 48, and SEQ ID Nos. 57 to 74
(R6) a nucleic acid having a base sequence obtained by deletion, substitution, addition, and/or insertion of one or more bases in the base sequence described in (R5)
(R7) a nucleic acid that hybridizes to a nucleic acid having a base sequence complementary to the base sequences described in (R5) and (R6) under a stringent condition

The forward primer can be a nucleic acid consisting of a base sequence indicated by any of SEQ ID Nos. 3 to 18, SEQ ID Nos. 35 to 41, SEQ ID Nos. 49 to 56, and SEQ ID No. 75, but can be a nucleic acid including the base sequence. The reverse primer can be a nucleic acid consisting of a base sequence indicated by any of SEQ ID Nos. 19 to 34, SEQ ID Nos. 42 to 48, and SEQ ID Nos. 57 to 74, but can be a nucleic acid including the base sequence.

The present invention further provides a kit including the primer set for determining whether or not a subject has developed spinal muscular atrophy and/or a risk of developing spinal muscular atrophy. The present invention can be a kit for determining whether or not a subject has developed spinal muscular atrophy and/or a risk of developing spinal muscular atrophy by lateral flow assay of a PCR product. The present invention can be a kit for determining whether or not a subject has developed spinal muscular atrophy and/or a risk of developing spinal muscular atrophy that can determine whether or not the subject is a spinal muscular atrophy carrier, and the kit can determine homozygous deletion of the SMN1 gene, heterozygous deletion of the SMN1 gene, and absence of deletion of the SMN1 gene in DNA included in a sample.

Furthermore, the present invention can be a method for determining whether or not a subject has developed spinal muscular atrophy and/or a risk of developing spinal muscular atrophy, including a PCR step of performing PCR by using the primer set. The PCR step may be non-quantitative PCR instead of quantitative PCR or real-time PCR. Furthermore, the method may be a method for determining whether or not a subject has developed spinal muscular atrophy and/or a risk of developing spinal muscular atrophy by lateral flow assay pf a PCR product obtained in the PCR step.

### ADVANTAGEOUS EFFECTS OF INVENTION

In PCR using the primer set of the present invention, the SMN1 gene is specifically amplified with high sensitivity, and therefore a result excluding influence of the SMN2 gene can be obtained. In real-time PCR using the primer set of the present invention, whether or not a subject (subject person) has developed SMA and a risk of developing SMA can be evaluated with extremely high accuracy, as compared with the conventional arts.

Furthermore, by applying the primer set of the present invention to an easy method such as electrophoretic analysis of an amplified product of non-quantitative PCR and lateral flow assay of a PCR product, whether or not a subject (subject person) has developed SMA and a risk of developing SMA can be evaluated. In this case, the determination of the present invention is advantageous over the conventional arts, for example, in that expensive equipment for real-time PCR is not needed or that a result can be obtained speedily and easily.

### BRIEF DESCRIPTION OF DRAWINGS

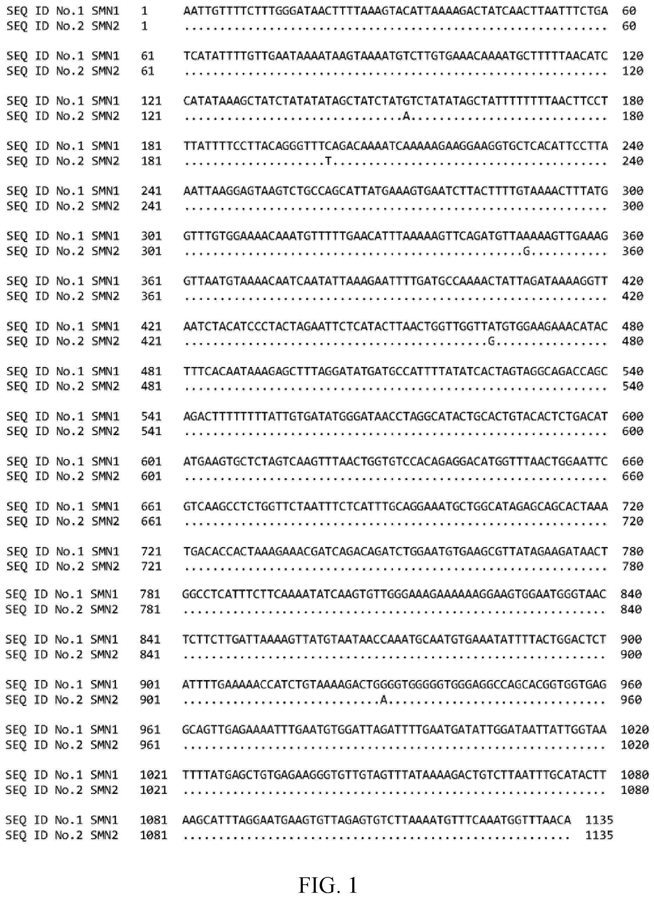

[Fig. 1] Fig. 1 illustrates a result of alignment between a partial base sequence (SEQ ID No. 1) of the SMN1 gene and a partial base sequence (SEQ ID No. 2) of the SMN2 gene. "·" in Fig. 1 indicates that a base on the SMN2 gene is identical to a base on the SMN1 gene as a result of the alignment.

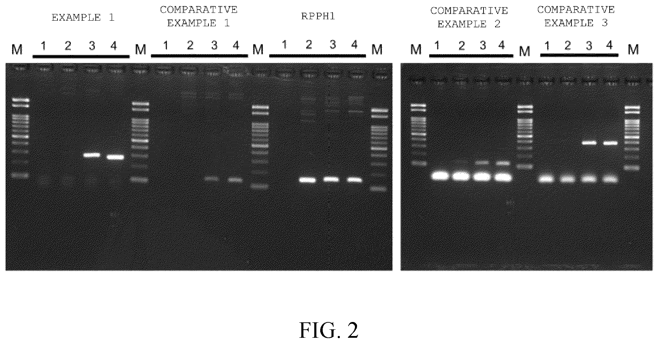

[Fig. 2] Fig. 2 illustrates an electrophoretic profile of an amplified product produced by non-quantitative PCR using the primer sets of Example 1 and Comparative Examples 1 to 3 of the present invention. In Fig. 2, a character M indicative of a lane is a marker, 1 indicates that negative contrast was used as a template, 2 indicates that a DNA sample with homozygous deletion was used as a template, 3 indicates that a DNA sample with heterozygous deletion was used as a template, and 4 indicates that a DNA sample of a healthy subject (without deletion) was used as a template.

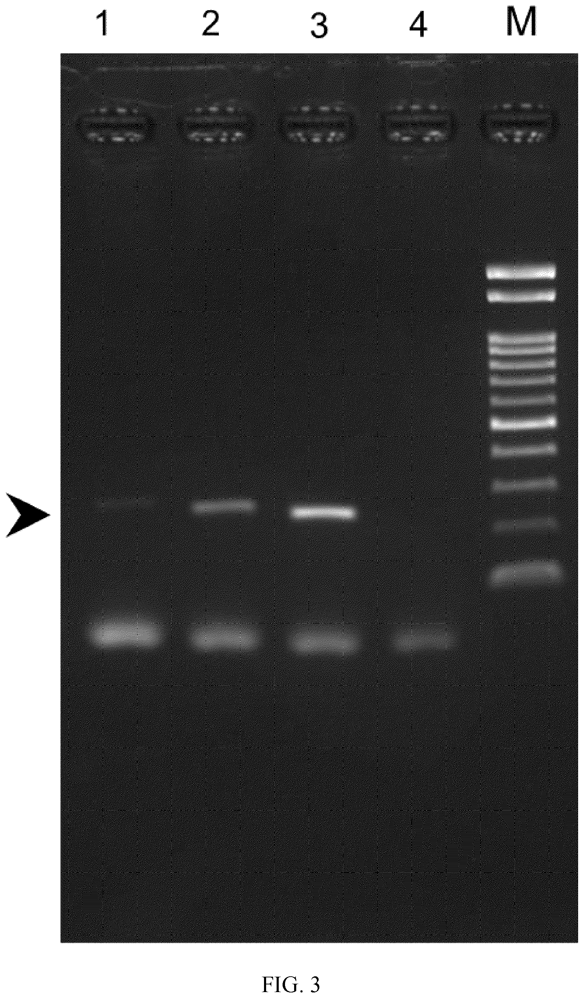

[Fig. 3] Fig. 3 illustrates an electrophoretic profile of an amplified product produced by non-quantitative PCR using primer set of Example of the present invention. In Fig. 3, a character M indicative of a lane is a marker, 1 indicates that a DNA sample with homozygous deletion was used as a template, 2 indicates that a DNA sample with heterozygous deletion was used as a template, 3 indicates that a DNA sample of a healthy subject (without deletion) was used as a template, and 4 indicates that nuclease-free water as negative contrast was used as a template. Among bands confirmed by electrophoresis, a band indicated by the arrowhead is a band derived from the SMN1 gene.

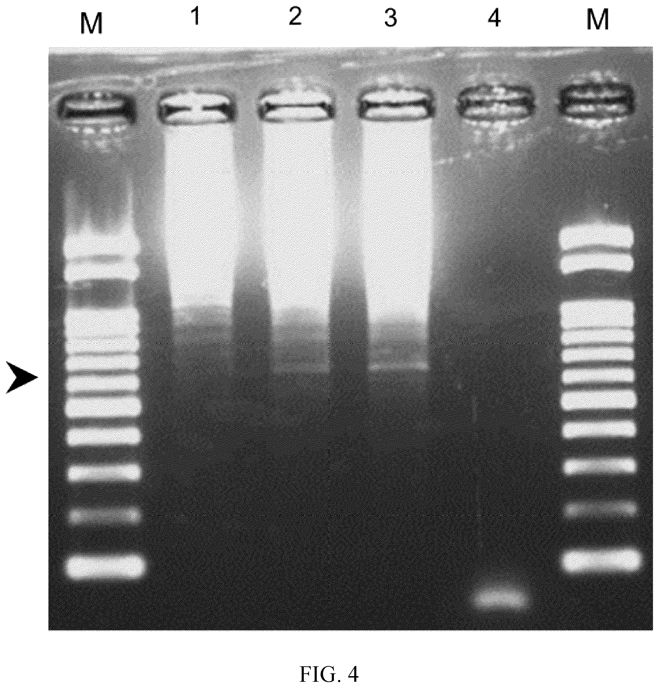

[Fig. 4] Fig. 4 illustrates an electrophoretic profile of an amplified product produced by non-quantitative PCR using primer set of Example of the present invention. In Fig. 4, a character M indicative of a lane is a marker, 1 indicates that a DNA sample with homozygous deletion was used as a template, 2 indicates that a DNA sample with heterozygous deletion was used as a template, 3 indicates that a DNA sample of a healthy subject (without deletion) was used as a template, and 4 indicates that nuclease-free water as negative contrast was used as a template. Among bands confirmed by electrophoresis, a band indicated by the arrowhead is a band derived from the SMN1 gene.

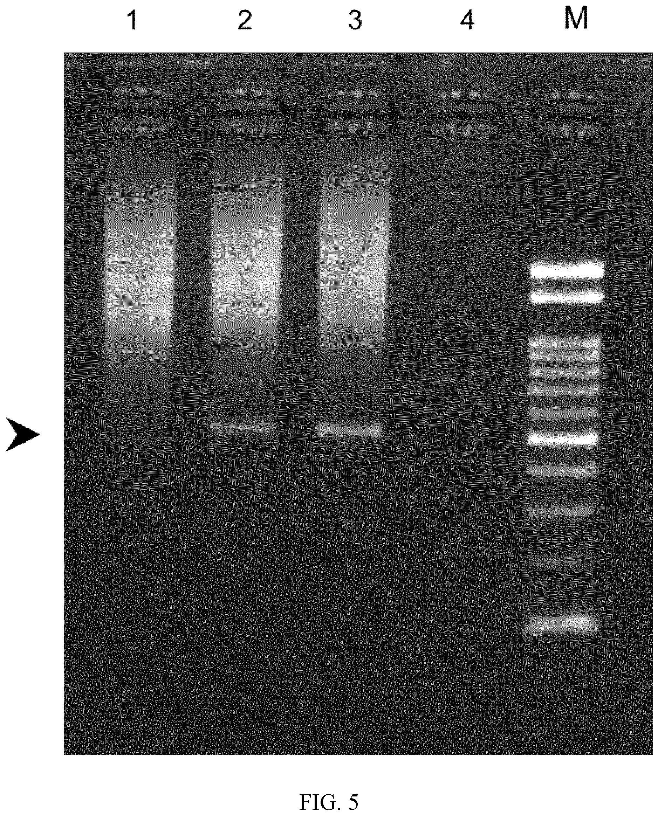

[Fig. 5] Fig. 5 illustrates an electrophoretic profile of an amplified product produced by non-quantitative PCR using primer set of Example of the present invention. In Fig. 5, a character M indicative of a lane is a marker, 1 indicates that a DNA sample with homozygous deletion was used as a template, 2 indicates that a DNA sample with heterozygous deletion was used as a template, 3 indicates that a DNA sample of a healthy subject (without deletion) was used as a template, and 4 indicates that nuclease-free water as negative contrast was used as a template. Among bands confirmed by electrophoresis, a band indicated by the arrowhead is a band derived from the SMN1 gene.

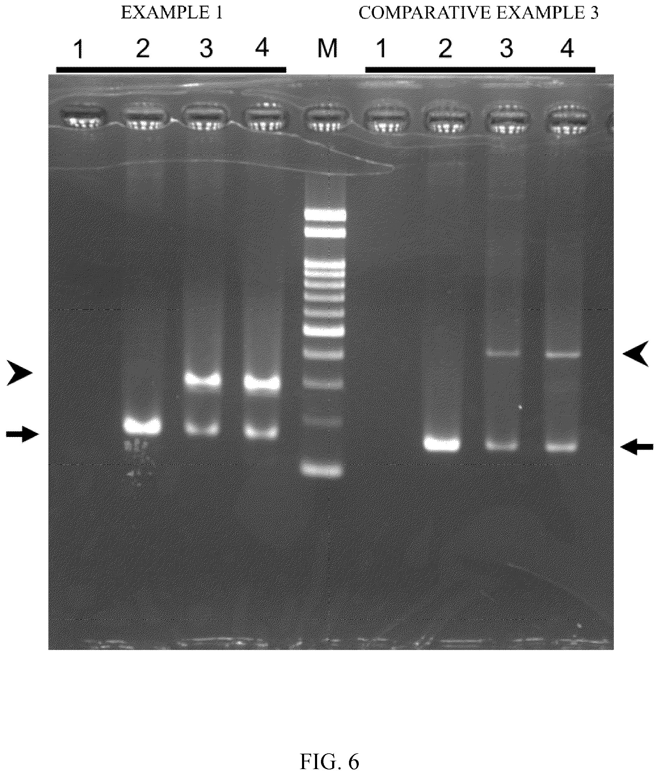

[Fig. 6] Fig. 6 illustrates an electrophoretic profile of an amplified product produced by non-quantitative PCR using primer sets of Example 1 and Comparative Example 3 of the present invention. In Fig. 6, a character M indicative of a lane is a marker, 1 indicates that negative contrast was used as a template, 2 indicates that a DNA sample with homozygous deletion was used as a template, 3 indicates that a DNA sample with heterozygous deletion was used as a template, and 4 indicates that a DNA sample of a healthy subject (without deletion) was used as a template. Among bands confirmed by electrophoresis, a band indicated by the arrowhead is a band derived from the SMN1 gene, and a band indicated by the arrow is a band derived from the RPPH1 gene.

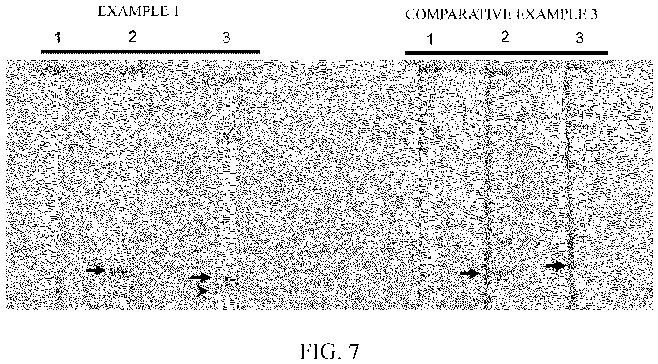

[Fig. 7] Fig. 7 is a photograph showing an analysis result of lateral flow assay of a PCR product using the primer sets of Example 1 and Comparative Example 3 of the present invention. 1 indicates that negative contrast was used as a template, 2 indicates that a DNA sample with homozygous deletion was used as a template, and 3 indicates that a DNA sample with heterozygous deletion was used as a template. Among lines formed on a strip, a line indicated by the arrowhead is a line formed by an amplified product derived from the SMN1 gene, and a line indicated by the arrow is a line formed by an amplified product derived from the RPPH1 gene.

### DESCRIPTION OF EMBODIMENTS

The present invention will be described in detail on the basis of embodiments, but the present invention is not limited to these embodiments.

The present invention is a primer set for determining whether or not a subject has developed spinal muscular atrophy and/or a risk of developing spinal muscular atrophy, in which the primer set amplifies a Survival motor neuron (SMN) 1 gene, and a forward primer and a reverse primer included in the primer set each have a base that hybridizes to a base on the SMN1 gene that differs in a case where the SMN1 gene is aligned with a SMN2 gene. Furthermore, the present invention provides a kit including the primer set for determining whether or not a subject has developed spinal muscular atrophy and/or a risk of developing spinal muscular atrophy and a method for determining whether or not a subject has developed spinal muscular atrophy and/or a risk of developing spinal muscular atrophy.

The primer set according to the present invention is a primer set that can specifically amplify the SMN1 gene. The expression "specifically amplify the SMN1 gene" refers to "amplify the SMN1 gene and hardly amplify the SMN2 gene or not amplify the SMN2 gene". That is, reactivity of the primer set according to the present invention to the SMN2 gene is remarkably lower than reactivity thereof to the SMN1 gene. Specifically, the reactivity of the primer set according to the present invention to the SMN2 gene can be less than 1%, less than 0.1%, or less than 0.01% of the reactivity to the SMN1 gene.

The primer set according to the present invention is designed so that 3'-end-side bases of the forward primer and the reverse primer bind to bases in the SMN1 gene that are different from the SMN2 gene. Specifically, the forward primer is a primer whose 3'-end-side base binds to any of bases located at c.835-44 (hereinafter, "c." means "coding DNA"), c.840, c.888+100, and c.888+215 on an antisense strand of the SMN1 gene, and the reverse primer is a primer whose 3'-end-side base binds to any of bases located at c.840, c.888+100, c.888+215, and c.1155 on a sense strand of the SMN1 gene. The primer set according to the present invention is a primer set including the forward primer and the reverse primer.

The 3'-end-side bases of the forward primer and the reverse primer of the primer set according to the present invention bind to the SMN1 gene but do not bind to the SMN2 gene. Therefore, in PCR using the primer set, the SMN2 gene is not amplified or is amplified with efficiency remarkably lower than the SMN1 gene. In the present invention, both the forward primer and the reverse primer have this base on the 3'-end side, and therefore a risk of non-specific amplification of the SMN2 gene is lowered. The PCR using the primer set according to the present invention can accurately determine the presence or absence of the SMN1 gene in a sample or an amount of the SMN1 gene present in the sample.

In other words, the present invention is a primer set including a forward primer and a reverse primer having a base that hybridizes to a base on the SMN1 gene that differs when the SMN1 gene is aligned with the SMN2 gene. Fig. 1 illustrates a result of aligning a partial sequence of the SMN1 gene and a partial sequence of the SMN2 gene by using ClustalW. The base sequence indicated by SEQ ID No. 1 is a partial sequence from the 31806th base to the 32940th base of the SMN1 gene (GeneBank Accession No.: NG_008691) on human chromosome 5 and includes exon 7 of the SMN1 gene. The base sequence indicated by SEQ ID No. 2 is a partial sequence from the 31804th base to the 32938th base of the SMN2 gene (GeneBank Accession No.: NG_008728) that has high homology with the base sequence indicated by SEQ ID No. 1.

In one embodiment of the primer set according to the present invention, the forward primer and the reverse primer have a base that hybridizes to a base on the SMN1 gene that differs from the SMN2 gene, that is, any of the 152nd base (corresponding to c.835-44), the 201st base (corresponding to c.840), the 349th base (corresponding to c.888+100), the 464th base (corresponding to c. 888+215), and the 929th base (corresponding to c.1155) of the base sequence indicated by SEQ ID No. 1.

More specifically, in one embodiment of the present invention, the base which the forward primer has hybridizes to a base complementary to any of the 152nd base, the 201st base, the 349th base, and the 464th base of the base sequence indicated by SEQ ID No. 1, and the base which the reverse primer has hybridizes to a base homologous to any of the 201st base, the 349th base, the 464th base, and the 929th base of the base sequence indicated by SEQ ID No. 1.

In one embodiment, the base that hybridizes is located on the 3'-end sides of the forward primer and the reverse primer. This is because a risk of amplifying the SMN2 gene is lowered in a case where the base is located on the 3'-end sides of the primers. In another embodiment, the base that hybridizes may be located at the first to fifth base from the 3'-end of each primer.

In one embodiment, the forward primer has, at the first, second, third, fourth, or fifth base from the 3' end, a base homologous to the 152nd, 201st, 349th, or 464th base of the base sequence indicated by SEQ ID No. 1. The forward primer has, in a 5' direction from the homologous base, a sequence consecutively homologous to a base sequence in a 5' direction of the base sequence indicated by SEQ ID No. 1. The reverse primer has, at the first, second, third, fourth, or fifth base from the 3' end, a base complementary to the 201st, 349th, 464th, or 929th base of the base sequence indicated by SEQ ID No. 1. The reverse primer has, in a 5' direction from the complementary base, a sequence consecutively complementary to the base sequence in a 3' direction of the base sequence indicated by SEQ ID No. 1.

A primer set according to another embodiment can be a combination of one or more primers among the above forward primers and reverse primers. The combination of primers according to the present embodiment can be a combination of a forward primer having a base homologous to the 152nd base of the base sequence indicated by SEQ ID No. 1 and a reverse primer having a base complementary to the 201st, 349th, 464th, or 929th base of the same base sequence, a combination of a forward primer having a base homologous to the 201st base of the base sequence indicated by SEQ ID No. 1 and a reverse primer having a base complementary to the 349th, 464th, or 929th base of the same base sequence, a combination of a forward primer having a base homologous to the 349th base of the base sequence indicated by SEQ ID No. 1 and a reverse primer having a base complementary to the 464th or 929th base of the same base sequence, or a combination of a forward primer having a base homologous to the 464th base of the base sequence indicated by SEQ ID No. 1 and a reverse primer having a base complementary to the 929th base of the same base sequence.

In another embodiment of the present invention, the forward primer and/or the reverse primer has a mismatch base that forms a mismatched base pair with the template SMN1 gene. In a case where a mismatched base pair is formed between the primer and the template, a risk of occurrence of non-specific amplification is further lowered. In the present invention, the forward primer and/or the reverse primer may include one or more mismatch bases although the number of mismatch bases on the primer is not limited as long as the SMN1 gene is amplified.

The mismatched base pair means that a base of a template and a base of a primer corresponding to the base are not complementary. For example, in a case where the base on the template is cytosine, the mismatch base on the primer is a base other than guanine, specifically, adenine, cytosine, or thymine. Furthermore, the mismatch base on the primer may be an artificial base that is not specifically paired with the base on the template.

In one embodiment, the mismatch base can be located on the 3'-end side of the forward primer and/or the reverse primer, more specifically, any of the first to sixth bases from the 3' end of each primer. In another aspect, the mismatch base on the primer can be a base adjacent to a base that hybridizes to a base on the SMN1 gene that differs when the SMN1 gene is aligned with the SMN2 gene. In this case, even if non-specific binding occurs between the primer and the SMN2 gene, occurrence of non-specific amplification can be prevented in a case where a mismatched base pair is formed between the base adjacent to the base that hybridizes and the template. The mismatch base on the forward primer and/or the reverse primer can be adjacent to the base that hybridizes in the 3' direction and/or 5' direction.

A specific example of the mismatch base is illustrated. In a forward primer having a base homologous to the 152nd base of the base sequence indicated by SEQ ID No. 1, the base is guanine (G). A base adjacent to the base on the forward primer in the 5' direction may be guanine (G), cytosine (C), or adenine (A) in a case where the primer has a mismatch base although the base is thymine (T) in a case where the primer has no mismatch base. Furthermore, a base adjacent to the base on the forward primer in the 3' direction may be guanine (G), cytosine (C), or adenine (A) in a case where the primer has a mismatch base although the base is thymine (T) in a case where the primer has no mismatch base.

In a forward primer having a base homologous to the 201st base of the base sequence indicated by SEQ ID No. 1, the base is cytosine (C). A base adjacent to the base on the forward primer in the 5' direction may be guanine (G), cytosine (C), or adenine (A) in a case where the primer has a mismatch base although the base is thymine (T) in a case where the primer has no mismatch base. Furthermore, a base adjacent to the base on the forward primer in the 3' direction may be guanine (G), cytosine (C), or thymine (T) in a case where the primer has a mismatch base although the base is adenine (A) in a case where the primer has no mismatch base.

In a forward primer having a base homologous to the 349th base of the base sequence indicated by SEQ ID No. 1, the base is adenine (A). A base adjacent to the base on the forward primer in the 5' direction may be guanine (G), cytosine (C), or thymine (T) in a case where the primer has a mismatch base although the base is adenine (A) in a case where the primer has no mismatch base. Furthermore, a base adjacent to the base on the forward primer in the 3' direction may be guanine (G), cytosine (C), or thymine (T) in a case where the primer has a mismatch base although the base is adenine (A) in a case where the primer has no mismatch base.

In a forward primer having a base homologous to the 464th base of the base sequence indicated by SEQ ID No. 1, the base is adenine (A). A base adjacent to the base on the forward primer in the 5' direction may be guanine (G), cytosine (C), or adenine (A) in a case where the primer has a mismatch base although the base is thymine (T) in a case where the primer has no mismatch base. Furthermore, a base adjacent to the base on the forward primer in the 3' direction may be guanine (G), cytosine (C), or adenine (A) in a case where the primer has a mismatch base although the base is thymine (T) in a case where the primer has no mismatch base.

In a reverse primer having a base complementary to the 201st base of the base sequence indicated by SEQ ID No. 1, the base is guanine (G). A base adjacent to the base on the reverse primer in the 5' direction may be guanine (G), cytosine (C), or adenine (A) in a case where the primer has a mismatch base although the base is thymine (T) in a case where the primer has no mismatch base. Furthermore, a base adjacent to the base on the reverse primer in the 3' direction may be guanine (G), cytosine (C), or thymine (T) in a case where the primer has a mismatch base although the base is adenine (A) in a case where the primer has no mismatch base.

In a reverse primer having a base complementary to the 349th base of the base sequence indicated by SEQ ID No. 1, the base is thymine (T). A base adjacent to the base on the reverse primer in the 5' direction may be guanine (G), cytosine (C), or adenine (A) in a case where the primer has a mismatch base although the base is thymine (T) in a case where the primer has no mismatch base. Furthermore, a base adjacent to the base on the reverse primer in the 3' direction may be guanine (G), cytosine (C), or adenine (A) in a case where the primer has a mismatch base although the base is thymine (T) in a case where the primer has no mismatch base.

In a reverse primer having a base complementary to the 464th base of the base sequence indicated by SEQ ID No. 1, the base is thymine (T). A base adjacent to the base on the reverse primer in the 5' direction may be guanine (G), cytosine (C), or thymine (T) in a case where the primer has a mismatch base although the base is adenine (A) in a case where the primer has no mismatch base. Furthermore, a base adjacent to the base on the reverse primer in the 3' direction may be guanine (G), cytosine (C), or thymine (T) in a case where the primer has a mismatch base although the base is adenine (A) in a case where the primer has no mismatch base.

In a reverse primer having a base complementary to the 929th base of the base sequence indicated by SEQ ID No. 1, the base is cytosine (C). A base adjacent to the base on the reverse primer in the 5' direction may be guanine (G), thymine (T), or adenine (A) in a case where the primer has a mismatch base although the base is cytosine (C) in a case where the primer has no mismatch base. Furthermore, a base adjacent to the base on the reverse primer in the 3' direction may be guanine (G), thymine (T), or adenine (A) in a case where the primer has a mismatch base although the base is cytosine (C) in a case where the primer has no mismatch base.

Base sequences of a forward primer and a reverse primer that can be included in the primer set according to the present invention are described in more detail by illustrating specific examples.

The base sequences of the forward primer described in Table 1 are examples of a base sequence that has, on the 3' end side, a base homologous to the 152nd base of the base sequence indicated by SEQ ID No. 1 and has, in the 5' direction from the base, a sequence consecutively homologous to a base sequence in the 5' direction from the 152nd base of the base sequence indicated by SEQ ID No. 1. The base sequences indicated by SEQ ID Nos. 3, 4, 5, and 6 have guanine (G), which is a base homologous to the 152nd base of the base sequence indicated by SEQ ID No. 1, at the first, second, third, and fourth bases from the 3' end, respectively. SEQ ID Nos. 4, 5, and 6 have, in the 3' direction from the base, a sequence consecutively homologous to a base sequence in the 3' direction from the 152nd base of the base sequence indicated by SEQ ID No. 1. Each of these base sequences has a length of 22 bases.

**[Table 1]**

| SEQ ID NO. | BASE SEQUENCE |
|---|---|
| 3 | TATCTATATATAGCTATCTATG |
| 4 | ATCTATATATAGCTATCTATGT |
| 5 | TCTATATATAGCTATCTATGTC |
| 6 | CTATATATAGCTATCTATGTCT |

In each of the above base sequences, a base adjacent to guanine (G) on the 3' end side may be a mismatch base. In this case, the second thymine (T) from the 3' end of SEQ ID No. 3, the third thymine (T) from the 3' end of SEQ ID No. 4, the fourth thymine (T) from the 3' end of SEQ ID No. 5, and the fifth thymine (T) from the 3' end of SEQ ID No. 6 can be adenine (A), guanine (G), and cytosine (C). Furthermore, the first thymine (T) from the 3' end of SEQ ID No. 4, the second thymine (T) from the 3' end of SEQ ID No. 5, and the third thymine (T) from the 3' end of SEQ ID No. 6 can be adenine (A), guanine (G), or cytosine (C).

The base sequences of the forward primer described in Table 2 are examples of a base sequence that has, on the 3' end side, a base homologous to the 201st base of the base sequence indicated by SEQ ID No. 1 and has, in the 5' direction from the base, a sequence consecutively homologous to a base sequence in the 5' direction from the 201st base of the base sequence indicated by SEQ ID No. 1. The base sequences indicated by SEQ ID Nos. 7, 8, 9, and 10 have cytosine (C), which is a base homologous to the 201st base of the base sequence indicated by SEQ ID No. 1, at the first, second, third, and fourth bases from the 3' end, respectively. SEQ ID Nos. 8, 9, and 10 have, in the 3' direction from the base, a sequence consecutively homologous to a base sequence in the 3' direction from the 201st base of the base sequence indicated by SEQ ID No. 1. Each of these base sequences has a length of 22 bases.

**[Table 2]**

| SEQ ID NO. | BASE SEQUENCE |
|---|---|
| 7 | TTTATTTTCCTTACAGGGTTTC |
| 8 | TTATTTTCCTTACAGGGTTTCA |
| 9 | TATTTTCCTTACAGGGTTTCAG |
| 10 | ATTTTCCTTACAGGGTTTCAGA |

In each of the above base sequences, a base adjacent to cytosine (C) on the 3' end side may be a mismatch base. In this case, the second thymine (T) from the 3' end of SEQ ID No. 7, the third thymine (T) from the 3' end of SEQ ID No. 8, the fourth thymine (T) from the 3' end of SEQ ID No. 9, and the fifth thymine (T) from the 3' end of SEQ ID No. 10 can be adenine (A), guanine (G), and cytosine (C). Furthermore, the first adenine (A) from the 3' end of SEQ ID No. 8, the second adenine (A) from the 3' end of SEQ ID No. 9, and the third adenine (A) from the 3' end of SEQ ID No. 10 can be thymine (T), guanine (G), or cytosine (C).

The base sequences of the forward primer described in Table 3 are examples of a base sequence that has, on the 3' end side, a base homologous to the 349th base of the base sequence indicated by SEQ ID No. 1 and has, in the 5' direction from the base, a sequence consecutively homologous to a base sequence in the 5' direction from the 349th base of the base sequence indicated by SEQ ID No. 1. The base sequences indicated by SEQ ID Nos. 11, 12, 13, and 14 have adenine (A), which is a base homologous to the 349th base of the base sequence indicated by SEQ ID No. 1, at the first, second, third, and fourth bases from the 3' end, respectively. SEQ ID Nos. 12, 13, and 14 have, in the 3' direction from the base, a sequence consecutively homologous to a base sequence in the 3' direction from the 349th base of the base sequence indicated by SEQ ID No. 1. Each of these base sequences has a length of 22 bases.

**[Table 3]**

| SEQ ID NO. | BASE SEQUENCE |
|---|---|
| 11 | ATTTAAAAAGTTCAGATGTTAA |
| 12 | TTTAAAAAGTTCAGATGTTAAA |
| 13 | TTAAAAAGTTCAGATGTTAAAA |
| 14 | TAAAAAGTTCAGATGTTAAAAA |

In each of the above base sequences, a base adjacent to adenine (A) on the 3' end side may be a mismatch base. In this case, the second adenine (A) from the 3' end of SEQ ID No. 11, the first and third adenine (A) from the 3' end of SEQ ID No. 12, the second and fourth adenine (A) from the 3' end of SEQ ID No. 13, and the third and fifth adenine (A) from the 3' end of SEQ ID No. 14 can be thymine (T), guanine (G), or cytosine (C).

The base sequences of the forward primer described in Table 4 are examples of a base sequence that has, on the 3' end side, a base homologous to the 464th base of the base sequence indicated by SEQ ID No. 1 and has, in the 5' direction from this base, a sequence consecutively homologous to a base sequence in the 5' direction from the 464th base of the base sequence indicated by SEQ ID No. 1. The base sequences indicated by SEQ ID Nos. 15, 16, 17, and 18 have adenine (A), which is a base homologous to the 464th base of the base sequence indicated by SEQ ID No. 1, at the first, second, third, and fourth bases from the 3' end, respectively. SEQ ID Nos. 16, 17, and 18 have, in the 3' direction from this base, a sequence consecutively homologous to the base sequence in the 3' direction from the 464th base of the base sequence indicated by SEQ ID No. 1. Each of these base sequences has a length of 22 bases.

**[Table 4]**

| SEQ ID NO. | BASE SEQUENCE |
|---|---|
| 15 | CTCATACTTAACTGGTTGGTTA |
| 16 | TCATACTTAACTGGTTGGTTAT |
| 17 | CATACTTAACTGGTTGGTTATG |
| 18 | ATACTTAACTGGTTGGTTATGT |

In each of the above base sequences, a base adjacent to adenine (A) on the 3' end side may be a mismatch base. In this case, the second thymine (T) from the 3' end of SEQ ID No. 15, the first and third thymine (T) from the 3' end of SEQ ID No. 16, the second and fourth thymine (T) from the 3' end of SEQ ID No. 17, and the third and fifth thymine (T) from the 3' end of SEQ ID No. 18 can be adenine (A), guanine (G), or cytosine (C).

The base sequences of the reverse primer described in Table 5 are examples of a base sequence that has, on the 3' end side, a base complementary to the 201st base of the base sequence indicated by SEQ ID No. 1 and has, in the 5' direction from this base, a sequence consecutively complementary to a base sequence in the 3' direction from the 201st base of the base sequence indicated by SEQ ID No. 1. The base sequences indicated by SEQ ID Nos. 19, 20, 21, and 22 have guanine (G), which is a base complementary to the 201st base of the base sequence indicated by SEQ ID No. 1, at the first, second, third, and fourth bases from the 3' end, respectively. SEQ ID Nos. 20, 21, and 22 have, in the 3' direction from the base, a sequence consecutively complementary to a base sequence in the 5' direction from the 201st base of the base sequence indicated by SEQ ID No. 1. Each of these base sequences has a length of 22 bases.

**[Table 5]**

| SEQ ID NO. | BASE SEQUENCE |
|---|---|
| 19 | TCCTTCTTTTTGATTTTGTCTG |
| 20 | CCTTCTTTTTGATTTTGTCTGA |
| 21 | CTTCTTTTTGATTTTGTCTGAA |
| 22 | TTCTTTTTGATTTTGTCTGAAA |

In each of the above base sequences, a base adjacent to guanine (G) on the 3' end side may be a mismatch base. In this case, the second thymine (T) from the 3' end of SEQ ID No. 19, the third thymine (T) from the 3' end of SEQ ID No. 20, the fourth thymine (T) from the 3' end of SEQ ID No. 21, and the fifth thymine (T) from the 3' end of SEQ ID No. 22 can be adenine (A), guanine (G), or cytosine (C). Furthermore, the first adenine (A) from the 3' end of SEQ ID No. 20, the second adenine (A) from the 3' end of SEQ ID No. 21, and the third adenine (A) from the 3' end of SEQ ID No. 22 can be thymine (T), guanine (G), or cytosine (C).

The base sequences of the reverse primer described in Table 6 are examples of a base sequence that has, on the 3' end side, a base complementary to the 349th base of the base sequence indicated by SEQ ID No. 1 and has, in the 5' direction from this base, a sequence consecutively complementary to the base sequence in the 3' direction from the 349th base of the base sequence indicated by SEQ ID No. 1. The base sequences indicated by SEQ ID Nos. 23, 24, 25, and 26 have thymine (T), which is a base complementary to the 349th base of the base sequence indicated by SEQ ID No. 1, at the first, second, third, and fourth bases from the 3' end, respectively. SEQ ID Nos. 24, 25, and 26 have, in the 3' direction from the base, a sequence consecutively homologous to a base sequence in the 5' direction from the 349th base of the base sequence indicated by SEQ ID No. 1. Each of these base sequences has a length of 22 bases.

**[Table 6]**

| SEQ ID NO. | BASE SEQUENCE |
|---|---|
| 23 | TTACATTAACCTTTCAACTTTT |
| 24 | TACATTAACCTTTCAACTTTTT |
| 25 | ACATTAACCTTTCAACTTTTTC |
| 26 | CATTAACCTTTCAACTTTTTCC |

In each of the above base sequences, a base adjacent to thymine (T) on the 3' end side may be a mismatch base. In this case, the second thymine (T) from the 3' end of SEQ ID No. 23, the first and third thymine (T) from the 3' end of SEQ ID No. 24, the second and fourth thymine (T) from the 3' end of SEQ ID No. 25, and the third and fifth thymine (T) from the 3' end of SEQ ID No. 26 can be adenine (A), guanine (G), or cytosine (C).

The base sequences of the reverse primer described in Table 7 are examples of a base sequence that has, on the 3' end side, a base complementary to the 464th base of the base sequence indicated by SEQ ID No. 1 and has, in the 5' direction from this base, a sequence consecutively complementary to a base sequence in the 3' direction from the 464th base of the base sequence indicated by SEQ ID No. 1. The base sequences indicated by SEQ ID Nos. 27, 28, 29, and 30 have thymine (T), which is a base complementary to the 464th base of the base sequence indicated by SEQ ID No. 1, at the first, second, third, and fourth bases from the 3' end, respectively. SEQ ID Nos. 28, 29, and 30 have, in the 3' direction from the base, a sequence consecutively complementary to a base sequence in the 5' direction from the 464th base of the base sequence indicated by SEQ ID No. 1. Each of these base sequences has a length of 22 bases.

**[Table 7]**

| SEQ ID NO. | BASE SEQUENCE |
|---|---|
| 27 | TGAAAGTATGTTTCTTCCACAT |
| 28 | GAAAGTATGTTTCTTCCACATA |
| 29 | AAAGTATGTTTCTTCCACATAA |
| 30 | AAGTATGTTTCTTCCACATAAC |

In each of the above base sequences, a base adjacent to thymine (T) on the 3' end side may be a mismatch base. In this case, the second adenine (A) from the 3' end of SEQ ID No. 27, the first and third adenine (A) from the 3' end of SEQ ID No. 28, the second and fourth adenine (A) from the 3' end of SEQ ID No. 29, and the third and fifth adenine (A) from the 3' end of SEQ ID No. 30 can be thymine (T), guanine (G), or cytosine (C).

The base sequences of the reverse primer described in Table 8 are examples of a base sequence that has, on the 3' end side, a base complementary to the 929th base of the base sequence indicated by SEQ ID No. 1 and has, in the 5' direction from the base, a sequence consecutively complementary to a base sequence in the 3' direction from the 929th base of the base sequence indicated by SEQ ID No. 1. The base sequences indicated by SEQ ID Nos. 31, 32, 33, and 34 have cytosine (C), which is a base complementary to the 929th base of the base sequence indicated by SEQ ID No. 1, at the first, second, third, and fourth bases from the 3' end, respectively. SEQ ID Nos. 32, 33, and 34 have, in the 3' direction from the base, a sequence consecutively complementary to a base sequence in the 5' direction from the 929th base of the base sequence indicated by SEQ ID No. 1. Each of these base sequences has a length of 22 bases.

**[Table 8]**

| SEQ ID NO. | BASE SEQUENCE |
|---|---|
| 31 | TGCTGGCCTCCCACCCCCACCC |
| 32 | GCTGGCCTCCCACCCCCACCCC |
| 33 | CTGGCCTCCCACCCCCACCCCA |
| 34 | TGGCCTCCCACCCCCACCCCAG |

In each of the above base sequences, a base adjacent to cytosine (C) on the 3' end side may be a mismatch base. In this case, the second cytosine (C) from the 3' end of SEQ ID No. 31, the first and third cytosine (C) from the 3' end of SEQ ID No. 32, the second and fourth cytosine (C) from the 3' end of SEQ ID No. 33, and the third and fifth cytosine (C) from the 3' end of SEQ ID No. 34 can be thymine (T), guanine (G), or adenine (A).

A specific example of the primer set according to the present invention is a combination of primers that can specifically amplify the SMN1 gene among the above forward primers and reverse primers. Examples include a combination of a forward primer having a base sequence indicated by any of SEQ ID Nos. 3 to 6 and a reverse primer having a base sequence indicated by any of SEQ ID Nos. 19 to 34, a combination of a forward primer having a base sequence indicated by any of SEQ ID Nos. 7 to 10 and a reverse primer having a base sequence indicated by any of SEQ ID Nos. 23 to 34, a combination of a forward primer having a base sequence indicated by any of SEQ ID Nos. 11 to 14 and a reverse primer having a base sequence indicated by any of SEQ ID Nos. 27 to 34, and a combination of a forward primer having a base sequence indicated by any of SEQ ID Nos. 15 to 18 and a reverse primer having a base sequence indicated by any of SEQ ID Nos. 31 to 34.

The present invention encompasses a nucleic acid having a base sequence indicated by any of SEQ ID Nos. described above, a nucleic acid having a base sequence indicated by SEQ ID Nos. described in Examples described later, and variants thereof that hybridize to nucleic acids having base sequences complementary to these nucleic acids under a stringent condition and have a function as a primer similar to a primer including these nucleic acids. The expression "hybridize under a stringent condition" is not limited specifically, and refers to, for example, keeping a hybridized state even after reaction for 1 to 24 hours in a solution containing SSC, a surfactant, and others at 30°C to 60°C and cleaning using a cleaning liquid containing SSC, SDS, and others.

The primers having a length of 22 bases have been illustrated as specific examples of the forward primer and the reverse primer included in the primer set according to the present invention. However, the base length of the primers is not limited at all as long as normal PCR reaction takes place, and can be a length of 8 to 40 bases, a length of 10 to 35 bases, a length of 15 to 33 bases, or a length of 20 to 30 bases in general.

More specifically, the base length of the primers can be a length of 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 bases. Even primers having a length of 7 bases or less and/or a length of 41 bases or more are encompassed within the present invention as long as normal PCR reaction takes place.

A person skilled in the art can select the base length of the primers as appropriate on the basis of a melting temperature (Tm value) or the like. A person skilled in the art can easily understand that a primer set including primers having any base length belongs to the present invention as long as the primer set specifically amplifies the SMN1 gene and does not amplify or hardly amplifies the SMN2 gene.

The forward primer and the reverse primer included in the primer set according to the present invention can be primers consisting of the base sequences described above in one example, but can be primers including the base sequences described above. In this case, the primer set can include any bases as long as the primer set specifically amplifies the SMN1 gene and does not amplify or hardly amplifies the SMN2 gene.

Furthermore, a person skilled in the art can easily understand that even a primer set including a forward primer and a reverse primer having base sequences obtained by deletion, substitution, addition, and/or insertion of one or more bases in the above base sequences of the primers belongs to the present invention, as long as the primer set specifically amplifies the SMN1 gene and does not amplify or hardly amplifies the SMN2 gene. The number of such bases is not limited, but specifically can be 2, 3, 4, 5, 6, 7, 8, 9, 10, or more.

The present invention further provides a kit for determining whether or not a subject has developed spinal muscular atrophy and/or a risk of developing spinal muscular atrophy. According to the kit according to the present invention, it is possible to determine whether DNA included in a sample is homozygous deletion of the SMN1 gene, heterozygous deletion of the SMN1 gene, or no deletion of the SMN1 gene.

The kit according to the present invention determines whether or not a subject has developed spinal muscular atrophy and/or a risk of developing spinal muscular atrophy by PCR lateral flow assay. The lateral flow assay is a method for determining the presence or absence and/or an amount of PCR product by capturing, at a predetermined position on a test strip, the PCR product that moves in a membrane strip by lateral flow, and is known as a speedy and easy determination method.

In the present invention, efficiency of amplification of the SMN1 gene is extremely high as compared with the primer sets described in the conventional art literatures, and therefore the kit including the primer set according to the present invention can perform the determination not only by real-time PCR, but also by electrophoretic analysis of a PCR product of non-quantitative PCR or lateral flow assay of the PCR product.

The present invention further provides a determination method. The present invention is a method for determining whether or not a subject has developed spinal muscular atrophy and/or a risk of developing spinal muscular atrophy including a step of performing PCR reaction by using the primer set by using a nucleic acid included in a specimen as a template and a step of determining the presence or absence of a PCR product or an amount of PCR. Preferably, the present invention is a method for determining whether or not a subject has developed spinal muscular atrophy and/or a risk of developing spinal muscular atrophy by lateral flow assay of a PCR product.

The determination method according to the present invention can be a determination method that can determine whether or not a subject person is a spinal muscular atrophy silent carrier or whether DNA included in a specimen is homozygous deletion of the SMN1 gene, heterozygous deletion of the SMN1 gene, or no deletion of the SMN1 gene. The determination method provided by the present invention can be a diagnostic method but may be a non-diagnostic method.

The subject in the present invention is a human or an animal. Examples of the animal include pet animals such as dogs and cats, farm animals such as cattle, pigs, sheep, goats, and chickens, and laboratory animals such as mice, rats, and rabbits. The sample used in the present invention is not limited as long as the sample is collected from a subject (subject person) and genomic DNA can be extracted therefrom, and specific examples thereof include urine, a nail, a hair, skin, saliva, and blood. The extraction of the genomic DNA from the sample can be performed by using a commercially available kit or the like.

### EXAMPLES

The present invention will be described in more detail with reference to Examples, but the present invention is not limited to Examples below.

### 1. Evaluation of Primer Sets of Example and Comparative Examples Using Real-Time PCR

A primer set of Example and primer sets described in the conventional art literatures (Comparative Examples 1 to 3) were evaluated by using QuantStudio (Registered Trademark) 5 real-time PCR system (ThermoFisher). Comparative Example 1 is a primer set described in Non Patent Literature 1, Comparative Example 2 is a primer set described in Non Patent Literature 2, and Comparative Example 3 is a primer set described in Non Patent Literature 3. A primer set that amplifies the Ribonuclease P RNA Component H1 (RPPH1) gene was used as an internal standard. Table 9 illustrates SEQ ID Nos. and base sequences of forward primers and reverse primers of the primer sets of Example 1 and Comparative Examples 1 to 3.

**[Table 9]**

| | FORWARD PRIMER | | REVERSE PRIMER | |
|---|---|---|---|---|
| | SEQ ID NO. | BASE SEQUENCE | SEQ ID NO. | BASE SEQUENCE |
| EXAMPLE 1 | 37 | TTCCTTTATTTTCCTTACAGG GTTCCAG | 45 | TTGTTTTACATTAACCTTTCAAC TTTT |
| COMPARATIV E EXAMPLE 1 | 76 | TATTTTCCTTACAGGGTTCC AG | 77 | GCTGGCAGACTTACTCCTTAAT TTAA |
| COMPARATIV E EXAMPLE 2 | 78 | ATAAAGCTATCTATATATATAG CTATCTATG | 79 | CCTTCTTTTTGATTTTGTCTG |
| COMPARATIV E EXAMPLE 3 | 80 | TTTATTTTCCTTACAGGGTTT C | 81 | GTGAAAGTATGTTTCTTCCACG TA |

As for template DNA, DNA derived from B cells of an SMA patient (Coriell Institute for Medical Research ID: NA23686) was used as a sample of the SMN1 gene with homozygous deletion, DNA derived from B cells of an SMA carrier (Coriell Institute for Medical Research ID: NA23687) was used as a sample of the SMN1 gene with heterozygous deletion, and genomic DNA derived from human adult normal tissues: peripheral blood leukocyte (Cosmo Bio Co., Ltd.) was used as a sample of the SMN1 gene without deletion. Nuclease-free water (Invitrogen) was used as negative contrast.

1 ng of the template DNA was added to a PCR reaction liquid containing 1x Taq Man Fast Advanced Master Mix (ThermoFisher), 0.2 µM of Taq Man Probe (ThermoFisher), 0.2 µM of a forward primer, and 0.2 µM of a reverse primer. PCR was conducted under two temperature conditions, specifically, a condition (hereinafter referred to as "60°C") of repeating 95°C for 15 seconds and 60°C for one minute forty times after 50°C for two minutes and 95°C for ten minutes and a condition (hereinafter referred to as "58°C") of repeating 95°C for 15 seconds and 58°C for one minute forty times after 50°C for two minutes and 95°C for ten minutes.

Table 10 illustrates a result of 60°C, and Table 11 illustrates a result of 58°C. The numerical values described in Table 10 and Table 11 are Fold-change assuming that amplification efficiency under a condition that the DNA sample without defective SMN1 gene was used as a template in Example 1 is "1.00". In Example 1, amplification was not confirmed as for the sample with homozygous deletion, but the sample with heterozygous deletion and the sample without deletion exhibited high amplification efficiency relative to Comparative Examples 1 to 3. In Comparative Example 2, amplification was not confirmed even in a case where the sample without deletion was used.

A difference in Fold-change between the sample with heterozygous deletion and the sample without deletion was less than 0.1 in Comparative Examples 1 and 3, whereas a difference of 0.33 to 0.43 was confirmed in Example 1. Therefore, according to real-time PCR using the primer set of Example 1, three kinds of samples, specifically, the sample with homozygous deletion, the sample with heterozygous deletion, and the sample without deletion could be easily distinguished.

**[Table 10]**

| | NEGATIVE CONTRAST | HOMOZYGOUS DELETION | HETEROZYGOUS DELETION | NO DELETION |
|---|---|---|---|---|
| EXAMPLE 1 | - | - | 0.57 | 1.00 |
| COMPARATIVE EXAMPLE 1 | - | - | 0.17 | 0.24 |
| COMPARATIVE EXAMPLE 2 | - | - | - | - |
| COMPARATIVE EXAMPLE 3 | - | - | 0.15 | 0.21 |

| | | | | |
|---|---|---|---|---|
| "-" indicates that a Ct value was indeterminable. | | | | |

**[Table 11]**

| | NEGATIVE CONTRAST | HOMOZYGOUS DELETION | HETEROZYGOUS DELETION | NO DELETION |
|---|---|---|---|---|
| EXAMPLE 1 | - | - | 0.67 | 1.00 |
| COMPARATIVE EXAMPLE 1 | - | - | 0.18 | 0.26 |
| COMPARATIVE EXAMPLE 2 | - | - | - | - |
| COMPARATIVE EXAMPLE 3 | - | - | 0.14 | 0.18 |

| | | | | |
|---|---|---|---|---|
| "-" indicates that a Ct value was indeterminable. | | | | |

### 2. Evaluation of Primer Set of Another Example Using Real-Time PCR (1)

Evaluation was conducted by using another primer set including any of the forward primers described in Table 12 and any of the reverse primers described in Table 12 by the same method as that described above. Each forward primer and each reverse primer described in Table 12 were designed to bind to c.840 and c.888+100 on the SMN1 gene, respectively, and have a length of 24 to 30 bases. In the forward primer, cytosine (C) that is a base that binds to c.840 on the SMN1 gene is located at the third position from the 3' end, and a base adjacent to this base on the 5' end side was changed from thymine (T) to cytosine (C).

**[Table 12]**

| FORWARD PRIMER | | REVERSE PRIMER | |
|---|---|---|---|
| SEQ ID NO. | BASE SEQUENCE | SEQ ID NO. | BASE SEQUENCE |
| 35 | ACTTCCTTTATTTTCCTTACAGGGTTCCAG | 42 | GGATTGTTTTACATTAACCTTTCAACTTT T |
| 36 | CTTCCTTTATTTTCCTTACAGGGTTCCAG | 43 | GATTGTTTTACATTAACCTTTCAACTTTT |
| 37 | TTCCTTTATTTTCCTTACAGGGTTCCAG | 44 | ATTGTTTTACATTAACCTTTCAACTTTT |
| 38 | TCCTTTATTTTCCTTACAGGGTTCCAG | 45 | TTGTTTTACATTAACCTTTCAACTTTT |
| 39 | CCTTTATTTTCCTTACAGGGTTCCAG | 46 | TGTTTTACATTAACCTTTCAACTTTT |
| 40 | CTTTATTTTCCTTACAGGGTTCCAG | 47 | GTTTTACATTAACCTTTCAACTTTT |
| 41 | TTTATTTTCCTTACAGGGTTCCAG | 48 | TTTTACATTAACCTTTCAACTTTT |

The same evaluation method as that described above was used, and the temperature condition was "60°C". Tables 13 to 19 show results. The numerical values described in the tables are Fold-change assuming that amplification efficiency under a condition that the DNA sample without defective SMN1 gene was used as a template in each primer set is "1.00". According to real-time PCR using any of the primer sets, three kinds of samples, specifically, the sample with homozygous deletion, the sample with heterozygous deletion, and the sample without deletion could be easily distinguished.

**[Table 13]**

| BASE SEQUENCE OF FORWARD PRIMER: SEQ ID NO. 35 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 42 | - | - | 0.64 | 1.00 |
| SEQ ID NO. 43 | - | 0.00 | 0.66 | 1.00 |
| SEQ ID NO. 44 | - | - | 0.64 | 1.00 |
| SEQ ID NO. 45 | - | - | 0.55 | 1.00 |
| SEQ ID NO. 46 | - | - | 0.51 | 1.00 |
| SEQ ID NO. 47 | - | - | 0.52 | 1.00 |

| | | | | |
|---|---|---|---|---|
| "-" indicates that a Ct value was indeterminable. | | | | |

**[Table 14]**

| BASE SEQUENCE OF FORWARD PRIMER: SEQ ID NO. 36 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 42 | - | 0.00 | 0.63 | 1.00 |
| SEQ ID NO. 43 | - | 0.00 | 0.49 | 1.00 |
| SEQ ID NO. 44 | - | - | 0.69 | 1.00 |
| SEQ ID NO. 45 | - | - | 0.51 | 1.00 |
| SEQ ID NO. 46 | - | - | 0.77 | 1.00 |
| SEQ ID NO. 47 | - | - | 0.56 | 1.00 |

| | | | | |
|---|---|---|---|---|
| "-" indicates that a Ct value was indeterminable. | | | | |

**[Table 15]**

| BASE SEQUENCE OF FORWARD PRIMER: SEQ ID NO. 37 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 42 | - | - | 0.83 | 1.00 |
| SEQ ID NO. 43 | - | 0.00 | 0.87 | 1.00 |
| SEQ ID NO. 44 | - | - | 0.31 | 1.00 |
| SEQ ID NO. 45 | - | - | 0.48 | 1.00 |
| SEQ ID NO. 46 | - | - | 0.90 | 1.00 |
| SEQ ID NO. 47 | - | - | 0.84 | 1.00 |

| | | | | |
|---|---|---|---|---|
| "-" indicates that a Ct value was indeterminable. | | | | |

**[Table 16]**

| BASE SEQUENCE OF FORWARD PRIMER: SEQ ID NO. 38 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 42 | - | - | 0.79 | 1.00 |
| SEQ ID NO. 43 | - | - | 0.57 | 1.00 |
| SEQ ID NO. 44 | - | - | 0.60 | 1.00 |
| SEQ ID NO. 45 | - | - | 0.66 | 1.00 |
| SEQ ID NO. 46 | - | - | 0.59 | 1.00 |
| SEQ ID NO. 47 | - | - | 0.58 | 1.00 |
| SEQ ID NO. 48 | - | - | 0.10 | 1.00 |

| | | | | |
|---|---|---|---|---|
| "-" indicates that a Ct value was indeterminable. | | | | |

**[Table 17]**

| BASE SEQUENCE OF FORWARD PRIMER: SEQ ID NO. 39 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 42 | - | - | 0.63 | 1.00 |
| SEQ ID NO. 43 | - | 0.00 | 0.73 | 1.00 |
| SEQ ID NO. 44 | - | - | 0.85 | 1.00 |
| SEQ ID NO. 45 | - | - | 0.60 | 1.00 |
| SEQ ID NO. 46 | - | - | 0.67 | 1.00 |
| SEQ ID NO. 47 | - | - | 0.33 | 1.00 |

| | | | | |
|---|---|---|---|---|
| "-" indicates that a Ct value was indeterminable. | | | | |

**[Table 18]**

| BASE SEQUENCE OF FORWARD PRIMER: SEQ ID NO. 40 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 42 | - | 0.01 | 0.51 | 1.00 |
| SEQ ID NO. 43 | - | 0.01 | 0.48 | 1.00 |
| SEQ ID NO. 44 | - | 0.01 | 0.52 | 1.00 |
| SEQ ID NO. 45 | - | 0.01 | 0.44 | 1.00 |
| SEQ ID NO. 46 | - | 0.00 | 0.59 | 1.00 |
| SEQ ID NO. 47 | - | - | 0.49 | 1.00 |

| | | | | |
|---|---|---|---|---|
| "-" indicates that a Ct value was indeterminable. | | | | |

**[Table 19]**

| BASE SEQUENCE OF FORWARD PRIMER: SEQ ID NO. 41 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 42 | - | - | 0.62 | 1.00 |
| SEQ ID NO. 43 | - | - | 0.66 | 1.00 |
| SEQ ID NO. 44 | - | - | 0.81 | 1.00 |
| SEQ ID NO. 45 | - | 0.03 | 0.73 | 1.00 |
| SEQ ID NO. 46 | - | - | 0.53 | 1.00 |
| SEQ ID NO. 47 | - | 0.06 | 0.82 | 1.00 |

| | | | | |
|---|---|---|---|---|
| "-" indicates that a Ct value was indeterminable. | | | | |

### 3. Evaluation Of Primer Set of Another Example Using Real-Time PCR (2)

Evaluation was conducted by using another primer set including any of the forward primers described in Table 20 and any of the reverse primers described in Table 20 by the same method as that described above. Each forward primer and each reverse primer described in Table 20 were designed to bind to c.840 and c.888+100 on the SMN1 gene, respectively.

**[Table 20]**

| FORWARD PRIMER | | REVERSE PRIMER | |
|---|---|---|---|
| SEQ ID NO. | BASE SEQUENCE | SEQ ID NO. | BASE SEQUENCE |
| 37 | TTCCTTTATTTTCCTTACAGGGTTCCAG | 45 | TTGTTTTACATTAACCTTTCAACTTTT |
| 49 | CTTCCTTTATTTTCCTTACAGGGTTCCA | 57 | TGTTTTACATTAACCTTTCAACTTTTT |
| 50 | ACTTCCTTTATTTTCCTTACAGGGTTCC | 58 | GTTTTACATTAACCTTTCAACTTTTTA |
| 51 | TTCCTTTATTTTCCTTACAGGGTTTCAG | 59 | TGTTTTACATTAACCTTTCAACTTTTA |
| 52 | CTTCCTTTATTTTCCTTACAGGGTTTCA | 60 | TTGTTTTACATTAACCTTTCAACTTCT |
| 53 | ACTTCCTTTATTTTCCTTACAGGGTTTC | 61 | TGTTTTACATTAACCTTTCAACTTCTT |
| 54 | TCCTTTATTTTCCTTACAGGGTTTCCGA | 62 | GTTTTACATTAACCTTTCAACTTCTTA |
| 55 | TTCCTTTATTTTCCTTACAGGGTTTCCG | | |
| 56 | CTTCCTTTATTTTCCTTACAGGGTTTCC | | |

The primers consisting of the base sequences indicated by SEQ ID Nos. 37, 49, and 50 were designed so that cytosine (C) that is a base that binds to c.840 on the SMN1 gene was located at the third, second, and first positions from the 3' end, and cytosine (C) that is a mismatch base was disposed at a position adjacent to this base on the 5' end side. The primers consisting of the base sequences indicated by SEQ ID Nos. 51 to 53 were obtained by replacing cytosine (C) that is a mismatch base on SEQ ID Nos. 37, 49, and 50 with thymine (T) that is not a mismatch base.

The primers consisting of the base sequences indicated by SEQ ID Nos. 54 to 56 were designed so that cytosine (C) that is a base that binds to c.840 on the SMN1 gene was located at the fourth, third, and second positions from the 3' end, and a base adjacent to this base on the 3' end side was changed from adenine (A) to cytosine (C) that is a mismatch base.

The primers consisting of the base sequences indicated by SEQ ID Nos. 45, 57, and 58 were designed so that thymine (T) that is a base that binds to c.888+100 on the SMN1 gene was located at the first, second, and third positions from the 3' end, and do not include a mismatch base. The primer consisting of the base sequence indicated by SEQ ID No. 59 was designed so that thymine (T) that is a base that binds to c.888+100 on the SMN1 gene was located at the second position from the 3' end, and a base adjacent to this base on the 3' end side was changed from thymine (T) to adenine (A) that is a mismatch base. The primers consisting of the base sequences indicated by SEQ ID Nos. 60 to 62 were obtained by changing a base adjacent to the above-mentioned thymine (T) of SEQ ID Nos. 45, 57, and 58 on the 5' end from thymine (T) to cytosine (C) that is a mismatch base.

The same evaluation method as that described above was used, and the temperature condition was "60°C". Tables 21 to 25 show results. The numerical values described in the tables are Fold-change assuming that amplification efficiency under a condition that the DNA sample without defective SMN1 gene was used as a template in each primer set is "1.00". According to real-time PCR using any of the primer sets, three kinds of samples, specifically, the sample with homozygous deletion, the sample with heterozygous deletion, and the sample without deletion could be easily distinguished.

**[Table 21]**

| BASE SEQUENCE OF FORWARD PRIMER: SEQ ID NO. 37 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 45 | - | - | 0.57 | 1.00 |
| SEQ ID NO. 57 | - | - | 0.63 | 1.00 |
| SEQ ID NO. 58 | - | - | 0.88 | 1.00 |
| SEQ ID NO. 59 | - | - | 0.63 | 1.00 |

| | | | | |
|---|---|---|---|---|
| "-" indicates that a Ct value was indeterminable. | | | | |

**[Table 22]**

| BASE SEQUENCE OF FORWARD PRIMER: SEQ ID NO. 49 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 45 | - | - | 0.74 | 1.00 |
| SEQ ID NO. 57 | - | - | 0.72 | 1.00 |
| SEQ ID NO. 58 | - | - | 0.35 | 1.00 |

| | | | | |
|---|---|---|---|---|
| "-" indicates that a Ct value was indeterminable. | | | | |

**[Table 23]**

| BASE SEQUENCE OF FORWARD PRIMER: SEQ ID NO. 50 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 45 | - | - | 0.72 | 1.00 |
| SEQ ID NO. 57 | - | 0.01 | 0.74 | 1.00 |
| SEQ ID NO. 58 | - | - | 0.69 | 1.00 |

| | | | | |
|---|---|---|---|---|
| "-" indicates that a Ct value was indeterminable. | | | | |

**[Table 24]**

| BASE SEQUENCE OF REVERSE PRIMER: SEQ ID NO. 45 | | | | |
|---|---|---|---|---|
| BASE SEQUENCE OF FORWARD PRIMER | NEGATIVE CONTRAST | HOMOZYGOUS DELETION | HETEROZYGOUS DELETION | NO DELETION |
| SEQ ID NO. 51 | - | 0.04 | 0.63 | 1.00 |
| SEQ ID NO. 52 | - | 0.07 | 0.72 | 1.00 |
| SEQ ID NO. 53 | - | 0.08 | 0.75 | 1.00 |
| SEQ ID NO. 54 | - | - | 0.34 | 1.00 |
| SEQ ID NO. 55 | - | - | 0.41 | 1.00 |
| SEQ ID NO. 56 | - | - | 0.31 | 1.00 |

| | | | | |
|---|---|---|---|---|
| "-" indicates that a Ct value was indeterminable. | | | | |

**[Table 25]**

| BASE SEQUENCE OF Fwd/Rev | NEGATIVE CONTRAST | HOMOZYGOUS DELETION | HETEROZYGOUS DELETION | NO DELETION |
|---|---|---|---|---|
| SEQ ID NO. 51/57 | - | 0.13 | 0.81 | 1.00 |
| SEQ ID NO. 52/57 | - | 0.23 | 0.86 | 1.00 |
| SEQ ID NO. 52/58 | - | 0.16 | 0.89 | 1.00 |
| SEQ ID NO. 53/58 | - | 0.11 | 0.33 | 1.00 |

| | | | | |
|---|---|---|---|---|
| "-" indicates that a Ct value was indeterminable. | | | | |

As for the following primer sets, ΔCT under conditions that the DNA sample with homozygous deletion and the DNA sample without deletion were used as templates is shown. According to real-time PCR using any of the primer sets, amplification enough to obtain ΔCT was not confirmed for the sample with homozygous deletion, and therefore the sample with homozygous deletion and the sample without deletion could be easily distinguished.

**[Table 26]**

| BASE SEQUENCE OF FORWARD PRIMER | BASE SEQUENCE OF REVERSE PRIMER | HOMOZYGOUS DELETION | NO DELETION |
|---|---|---|---|
| SEQ ID NO. 37 | SEQ ID NO. 60 | - | 4.7 |
| SEQ ID NO. 37 | SEQ ID NO. 61 | - | 4.5 |
| SEQ ID NO. 37 | SEQ ID NO. 62 | - | 4.5 |
| SEQ ID NO. 49 | SEQ ID NO. 60 | - | 4.7 |
| SEQ ID NO. 49 | SEQ ID NO. 61 | - | 4.4 |
| SEQ ID NO. 49 | SEQ ID NO. 62 | - | 4.2 |
| SEQ ID NO. 50 | SEQ ID NO. 60 | - | 4.6 |
| SEQ ID NO. 50 | SEQ ID NO. 61 | - | 4.2 |
| SEQ ID NO. 50 | SEQ ID NO. 62 | - | 4.2 |

| | | | |
|---|---|---|---|
| "-" indicates that a Ct value was indeterminable. | | | |

### Example 4, Evaluation of Primer Set of Another Example Using Real-Time PCR (3)

Evaluation was conducted by using another primer set including any of the reverse primers described in Table 27 by the same method as that described above. The primers described in Table 27 are reverse primers, and the primers indicated by SEQ ID Nos. 63 to 71 were designed to bind to c.888+215 on the SMN1 gene, and the primers indicated by SEQ ID Nos. 72 and 73 were designed to bind to c.1155 on the SMN1 gene. As a forward primer, a primer consisting of the base sequence indicated by SEQ ID No. 5 was used. The forward primer was designed to bind to c.840 on the SMN1 gene.

The primers indicated by SEQ ID Nos. 63 to 65 were designed so that the second thymine (T) from the 3' end bind to c.888+215 on the SMN1 gene. A base length of these primers is 24 to 26 bases. The primers indicated by SEQ ID Nos. 66 to 68 were designed so that thymine (T) located at the 3' end bind to c.888+215 on the SMN1 gene and have a length of 24 to 26 bases. In SEQ ID Nos. 66 to 68, a base adjacent to the thymine (T) that binds to c.888 + 215 on the SMN1 gene on the 5' end side is adenine (A). The primers indicated by SEQ ID Nos. 69 to 71 were obtained by replacing the adenine (A) on the SEQ ID Nos. 66 to 68 with guanine (G) so as to form a mismatched base pair with the SMN1 gene.

The primer indicated by SEQ ID No. 72 was designed so that cytosine (C) located at the 3' end binds to c.1155 on the SMN1 gene and a base adjacent to the cytosine (C) on the 5' end side is cytosine (C). The primer indicated by SEQ ID No. 73 was obtained by replacing the cytosine (C) on SEQ ID No. 72 with thymine (T) so as to form a mismatched base pair with the SMN1 gene.

**[Table 27]**

| SEQ ID NO | BASE SEQUENCE | SEQ ID NO | BASE SEQUENCE |
|---|---|---|---|
| 63 | GTGAAAGTATGTTTCTTCCACATA | 69 | TGTGAAAGTATGTTTCTTCCACGT |
| 64 | TGTGAAAGTATGTTTCTTCCACATA | 70 | TTGTGAAAGTATGTTTCTTCCACGT |
| 65 | TTGTGAAAGTATGTTTCTTCCACAT | 71 | ATTGTGAAAGTATGTTTCTTCCACG |
| | A | | T |
| 66 | TGTGAAAGTATGTTTCTTCCACAT | 72 | CGTGCTGGCCTCCCACCCCCACCC |
| 67 | TTGTGAAAGTATGTTTCTTCCACAT | 73 | CGTGCTGGCCTCCCACCCCCACTC |
| 68 | ATTGTGAAAGTATGTTTCTTCCACA T | | |

The same evaluation method as that described above was used, and the temperature condition was "60°C". Tables 28 and 29 show results. The numerical values described in the tables are Fold-change assuming that amplification efficiency under a condition that the DNA sample without defective SMN1 gene was used as a template in each primer set is "1.00". According to real-time PCR using any of the primer sets, three kinds of samples, specifically, the sample with homozygous deletion, the sample with heterozygous deletion, and the sample without deletion could be easily distinguished.

**[Table 28]**

| BASE SEQUENCE OF FORWARD PRIMER: SEQ ID NO. 37 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 63 | - | - | 0.80 | 1.00 |
| SEQ ID NO. 64 | - | - | 0.85 | 1.00 |
| SEQ ID NO. 65 | - | - | 0.72 | 1.00 |
| SEQ ID NO. 66 | - | - | 0.67 | 1.00 |
| SEQ ID NO. 67 | - | - | 0.81 | 1.00 |
| SEQ ID NO. 68 | - | - | 0.86 | 1.00 |
| SEQ ID NO. 69 | - | - | 0.74 | 1.00 |
| SEQ ID NO. 70 | - | - | 0.60 | 1.00 |
| SEQ ID NO. 71 | - | - | 0.92 | 1.00 |

| | | | | |
|---|---|---|---|---|
| "-" indicates that a Ct value was indeterminable. | | | | |

**[Table 29]**

| BASE SEQUENCE OF FORWARD PRIMER: SEQ ID NO. 37 | | | | |
|---|---|---|---|---|
| BASE SEQUENCE OF REVERSE PRIMER | NEGATIVE CONTRAST | HOMOZYGOUS DELETION | HETEROZYGOUS DELETION | NO DELETION |
| SEQ ID NO. 72 | - | - | 0.62 | 1.00 |
| SEQ ID NO. 73 | - | - | 0.72 | 1.00 |

| | | | | |
|---|---|---|---|---|
| "-" indicates that a Ct value was indeterminable. | | | | |

### 5. Evaluation of Primer Sets of Example and Comparative Examples Using Non-Quantitative PCR

Next, whether or not deletion of the SMN1 gene could be determined was verified by using an electrophoretic profile of an amplified product obtained by non-quantitative PCR. A PCR reaction liquid containing 0.025 U/µL of Taq DNA polymerase (Takara Bio Inc.), 0.5 µM of the primer set (Example 1 and Comparative Examples 1 to 3), and a predetermined buffer was produced, and the same DNA sample as 1. was used as a template. PCR was performed by using ProFlex (Registered Trademark) PCR System (ThermoFisher) under a temperature condition of repeating 98°C for 10 seconds, 60°C for 30 seconds, and 72°C for one minute thirty times after 98°C for 10 seconds and then setting the temperature to 4°C. The obtained PCR product was subjected to electrophoresis for 25 minutes at 100 V by using 2 weight% agarose gel.

Fig. 2 illustrates an electrophoretic profile. In Example 1 and Comparative Example 3, an amplified product was confirmed in a case where DNA with heterozygous deletion and DNA of a healthy subject (no deletion) were used as a template, and no amplified product was confirmed in a case where DNA with homozygous deletion was used as a template. In Comparative Example 1, an amount of amplified product was smaller than in Example 1 and Comparative Example 3. In Comparative Example 2, an amplified product was confirmed even in a case where DNA with homozygous deletion was used as a template. This shows that the primer sets of Example 1 and Comparative Example 3 can distinguish homozygous deletion of SMN1 from heterozygous deletion and no deletion by non-quantitative PCR.

Furthermore, evaluation was performed by using another primer set including any of the forward primers described in Table 30 and any of the reverse primers described in Table 30 by the same method as that described above. Among the primers described in Table 30, SEQ ID No. 74 is a forward primer designed to bind to c.888+100 on the SMN1 gene, and SEQ ID No. 75 is a forward primer designed to bind to c.888+215 on the SMN1 gene. The SEQ ID No. 68 is a reverse primer designed to bind to c.888+215 on the SMN1 gene, and the SEQ ID No. 72 is a reverse primer designed to bind to c.1155 on the SMN1 gene.

**[Table 30]**

| FORWARD PRIMER | | REVERSE PRIMER | |
|---|---|---|---|
| SEQ ID NO. | BASE SEQUENCE | SEQ ID NO. | BASE SEQUENCE |
| 7 4 | ACATTTAAAAAGTTCAGATGTTAA | 6 8 | ATTGTGAAAGTATGTTTCTTCCACAT |
| 7 5 | TCTCATACTTAACTGGTTGGTGAT | 7 2 | CGTGCTGGCCTCCCACCCCCACCC |

Fig. 3 illustrates an electrophoretic profile of a PCR amplified product using the primer set including the forward primer of SEQ ID No. 74 and the reverse primer of SEQ ID No. 68, Fig. 4 illustrates an electrophoretic profile of a PCR amplified product using the primer set including the forward primer of SEQ ID No. 75 and the reverse primer of SEQ ID No. 72, and Fig. 5 illustrates an electrophoretic profile of a PCR amplified product using the primer set including the forward primer of SEQ ID No. 74 and the reverse primer of SEQ ID No. 72. In all of the primer sets, no band was observed or only an extremely thin band was observed in a case where homozygous deletion was used as a template. In a case where heterozygous deletion was used as a template, a band was observed, but the band was thinner than that in a case where a sample without deletion was used as a template. Therefore, according to PCR and electrophoresis using any of the primer sets, three kinds of samples, specifically, the sample with homozygous deletion, the sample with heterozygous deletion, and the sample without deletion could be easily distinguished.

### 6. Evaluation of primer sets of Example and Comparative Example by lateral flow assay

Whether or not deletion of the SMN1 gene could be evaluated by the primer set of Example 1 and the primer set of Comparative Example 3 by using lateral flow assay, which is a speedy and easy determination method, was verified. The lateral flow was verified by using the Single-stranded Tag Hybridization method of TBA Co., Ltd. Specifically, according to this method, PCR reaction is performed with a primer labelled with biotin and single-tag DNA, a PCR product is mixed with avidin target latex and developed on a membrane strip, and the PCR product bound to the avidin coated latex is bound to complementary tag DNA solidified on the membrane, and thereby a line is formed on the membrane.

A PCR reaction liquid containing 0.025 U/µL of Taq DNA polymerase (Takara Bio Inc.), a labelled primer set of Example 1 or Comparative Example 3, a labelled primer set that amplifies the RPPH1 gene, and a predetermined buffer was produced, and the same DNA sample as 1. was used as a template. PCR reaction was performed by using ProFlex (Registered Trademark) PCR System (ThermoFisher), and the obtained PCR product was subjected to electrophoresis for 25 minutes at 100 V by using 2 weight% agarose gel.
In the lateral flow assay, the labelled PCR product was developed on a membrane strip in accordance with the Single-stranded Tag Hybridization method, and whether or not a line was formed was visually determined.

Fig. 6 illustrates an electrophoretic profile. Although a band derived from the SMN1 gene and a band derived from the RPPH1 gene were confirmed in lanes 3 (heterozygous deletion) and 4 (no deletion) in Example 1 and Comparative Example 3, the band derived from the SMN1 gene is darker in Example 1 than in Comparative Example 3. This shows that the SMN1 gene was efficiently amplified in Example 1. Furthermore, the band in the lane 4 (no deletion) is darker than the band in lane 3 (heterozygous deletion) in Example 1. This shows that an amount of SMN1 gene in a specimen can be estimated from an amplification amount.

Fig. 7 illustrates a photograph of the membrane strip. In Example 1, a line derived from the SMN1 gene and a line derived from the RPPH1 gene were formed on a strip (3 in Fig. 7) on which a PCR product obtained by using a DNA sample with heterozygous deletion as a template was developed. On the other hand, in Comparative Example 3, a line was formed by an amplified product derived from RPPH1, but a line derived from the SMN1 gene was not formed. This shows that whether or not the SMN1 gene is present in a sample can be determined by lateral flow assay in a case where the primer set of Example 1 is used although whether or not the SMN1 gene is present in a sample cannot be determined by lateral flow assay in a case where the primer set of Comparative Example is used.

In the present Example, it has been demonstrated that the primer set according to the present invention consisting of a specific base sequence is a primer set for determining whether or not a subject has developed spinal muscular atrophy and/or a risk of developing spinal muscular atrophy. Even a primer set including a primer having a base sequence other than the base sequence disclosed in the present Example can be encompassed within the present invention without departing from the technical idea of the present disclosure.

## Claims

1. A primer set for determining whether or not a subject has developed spinal muscular atrophy and/or a risk of developing spinal muscular atrophy, wherein
the primer set amplifies a Survival motor neuron (SMN) 1 gene, and
a forward primer and a reverse primer included in the primer set each have a base that hybridizes to a base on the SMN1 gene that differs in a case where the SMN1 gene is aligned with a SMN2 gene.

2. The primer set according to claim 1, wherein
the bases of the forward primer and the reverse primer are located on 3' end sides of the forward primer and the reverse primer.

3. The primer set according to claim 1, wherein
the bases of the forward primer and the reverse primer are located at any of first to fifth positions from 3' ends of the forward primer and the reverse primer.

4. The primer set according to claim 3, wherein
the base of the forward primer hybridizes to a base complementary to any of 152nd, 201st, 349th, and 464th bases of a base sequence indicated by SEQ ID No. 1, and
the base of the reverse primer hybridizes to a base homologous to any of the 201st, 349th, 464th, and 929th bases of the base sequence indicated by SEQ ID No. 1.

5. The primer set according to claim 1, wherein
the forward primer is any of (F1) to (F4), and the reverse primer is any of (R1) to (R4).
(F1) a primer that has, at any of first to fifth positions from a 3' end, a base homologous to a 152nd base of a base sequence indicated by SEQ ID No. 1 and has, in a 5' direction from the base, a sequence consecutively homologous to a base sequence in a 5' direction from the 152nd base of the base sequence indicated by SEQ ID No. 1
(F2) a primer that has, at any of first to fifth positions from a 3' end, a base homologous to a 201st base of the base sequence indicated by SEQ ID No. 1 and has, in a 5' direction from the base, a sequence consecutively homologous to a base sequence in the 5' direction from the 201st base of the base sequence indicated by SEQ ID No. 1
(F3) a primer that has, at any of first to fifth positions from a 3' end, a base homologous to a 349th base of the base sequence indicated by SEQ ID No. 1 and has, in a 5' direction from the base, a sequence consecutively homologous to a base sequence in the 5' direction from the 349th base of the base sequence indicated by SEQ ID No. 1
(F4) a primer that has, at any of first to fifth positions from a 3' end, a base homologous to a 464th base of the base sequence indicated by SEQ ID No. 1 and has, in a 5' direction from the base, a sequence consecutively homologous to a base sequence in the 5' direction from the 464th base of the base sequence indicated by SEQ ID No. 1
(R1) a primer that has, at any of first to fifth positions from a 3' end, a base complementary to the 201st base of the base sequence indicated by SEQ ID No. 1 and has, in a 5' direction from the base, a sequence consecutively complementary to a base sequence in a 3' direction from the 201st base of the base sequence indicated by SEQ ID No. 1
(R2) a primer that has, at any of first to fifth positions from a 3' end, a base complementary to the 349th base of the base sequence indicated by SEQ ID No. 1 and has, in a 5' direction from the base, a sequence consecutively complementary to a base sequence in the 3' direction from the 349th base of the base sequence indicated by SEQ ID No. 1
(R3) a primer that has, at any of first to fifth positions from a 3' end, a base complementary to the 464th base of the base sequence indicated by SEQ ID No. 1 and has, in a 5' direction from the base, a sequence consecutively complementary to a base sequence in the 3' direction from the 464th base of the base sequence indicated by SEQ ID No. 1
(R4) a primer that has, at any of first to fifth positions from a 3' end, a base complementary to a 929th base of the base sequence indicated by SEQ ID No. 1 and has, in a 5' direction from the base, a sequence consecutively complementary to a base sequence in the 3' direction from the 929th base of the base sequence indicated by SEQ ID No. 1

6. The primer set according to claim 5, wherein
the forward primer and the reverse primer are one or more selected from the group consisting of the following (a) to (d).
(a) the forward primer is the (F1), and the reverse primer is any of the (R1) to (R4)
(b) the forward primer is the (F2), and the reverse primer is any of the (R2) to (R4)
(c) the forward primer is the (F3), and the reverse primer is the (R3) or the (R4)
(d) the forward primer is the (F4), and the reverse primer is the (R4)

7. The primer set according to claim 3, wherein
the forward primer and/or the reverse primer have a mismatch base that forms a mismatched base pair with the SMN1 gene.

8. The primer set according to claim 7, wherein
the mismatch base is adjacent to the base that hybridizes to the base on the SMN1 gene that differs in a case where the SMN1 gene is aligned with the SMN2 gene.

9. The primer set according to any one of claims 1 to 8, wherein
the primer set specifically amplifies the SMN1 gene.

10. The primer set according to claim 9, wherein
the primer set does not amplify the SMN2 gene or hardly amplifies the SMN2 gene.

11. The primer set according to claim 10, wherein
the forward primer and the reverse primer have a length of 8 to 40 bases.

12. The primer set according to claim 10, wherein
the forward primer is any of (F5) to (F7), and the reverse primer is any of (R5) to (R7).
(R5) a nucleic acid having a base sequence indicated by any of SEQ ID Nos. 3 to 18, SEQ ID Nos. 35 to 41, SEQ ID Nos. 49 to 56, and SEQ ID No. 75
(R6) a nucleic acid having a base sequence obtained by deletion, substitution, addition, and/or insertion of one or more bases in the base sequence described in (F5)
(F7) a nucleic acid that hybridizes to a nucleic acid having a base sequence complementary to the base sequences described in (F5) and (F6) under a stringent condition
(R5) a nucleic acid having a base sequence indicated by any of SEQ ID Nos. 19 to 34, SEQ ID Nos. 42 to 48, and SEQ ID Nos. 57 to 74
(R6) a nucleic acid having a base sequence obtained by deletion, substitution, addition, and/or insertion of one or more bases in the base sequence described in (R5)
(R7) a nucleic acid that hybridizes to a nucleic acid having a base sequence complementary to the base sequences described in (R5) and (R6) under a stringent condition

13. A kit for determining whether or not a subject has developed spinal muscular atrophy and/or a risk of developing spinal muscular atrophy, the kit comprising the primer set according to claim 1.

14. The kit according to claim 13, wherein
the kit performs the determination by lateral flow assay of a PCR product.

15. The kit according to claim 13 or 14, wherein
the kit is capable of determining homozygous deletion of the SMN1 gene, heterozygous deletion of the SMN1 gene, and absence of deletion of the SMN1 gene in DNA included in a sample.

16. A method for determining whether or not a subject has developed spinal muscular atrophy and/or a risk of developing spinal muscular atrophy, the method comprising a PCR step of performing PCR by using the primer set according to claim 1.

17. The method according to claim 16, wherein
the PCR step is non-quantitative PCR.

18. The method according to claim 16 or 17, wherein
the determination is performed by lateral flow assay pf a PCR product obtained in the PCR step.
